# EUROPÄISCHE PATENTSCHRIFT

(11) **EP 0 557 546 B1**
(45) Veröffentlichungstag und Bekanntmachung des Hinweises auf die Patenterteilung: **07.05.1997**
(21) Anmeldenummer: 92103185.2
(22) Anmeldetag: 25.02.1992
(51) Int. Cl.: G01N 33/555, G01N 33/532, G01N 33/76, G01N 33/80

(54) **Kopplung von Antigenen und Antikörpern an nicht-fixierte Erythrozyten**
Binding of antigens and antibodies to non-fixed erythrocytes
Liaison des antigènes et des anticorps aux érythrocytes non-fixés

(43) Veröffentlichungstag der Anmeldung: 01.09.1993
(73) Patentinhaber: STIFTUNG FÜR DIAGNOSTISCHE FORSCHUNG, CH-1785 Cressier sur Morat (CH)
(72) Erfinder: Lapierre, Ives, F-69600 Oullins (FR); Josef, Dieter, CH-1785 Cressier (FR); Adam, Jean-R., CH-1884 Villars (FR); Greber-Widmer, Susanne, CH-3037 Herrenschwanden (FR)
(74) Vertreter: Huber, Bernhard, Dipl.-Chem.

(56) Entgegenhaltungen:
- EP-A- 0 074 271
- EP-A- 0 194 212
- WO-A-82/00203
- WO-A-87/04628
- FR-A- 1 351 642
- WORLD PATENTS INDEX LATEST, AN 90-344171, Week 9046, Derwent Publications Ltd., London,GB

## Beschreibung

Die vorliegende Erfindung betrifft ein Verfahren zur Bestimmung eines Analyten in einer wäßrigen Probelösung durch Agglutination von nicht-fixierten Erythrozyten.

Agglutinationstests, bei denen Antigene oder Antikörper an fixierte Erythrozyten gebunden werden, sind seit längerem bekannt. Durch das Fixieren wird dabei die Zellmembran des Erythrozyten stabilisiert, so daß die Zellen nicht mehr hämolysieren können. Werden nun solche beladenen Zellen mit dem entsprechenden Antikörper bzw. Antigen in Kontakt gebracht, so findet eine Agglutination statt.

Dieses Testverfahren stellt an sich eine sehr einfache und schnelle Bestimmungsmethode für Analyten in einer wäßrigen Probelösung dar. Es hat jedoch in den letzten Jahren stark an Bedeutung verloren, da es störanfällig, in Grenzfällen schwer zu interpretieren und im Vergleich zu neueren Methoden zu wenig empfindlich ist.

EP-A-0 074 271 beschreibt einen immunologischen Agglutinationstest, bei dem ein für ein Antigen spezifisches Immunglobulin an die Oberfläche eines Indikatorteilchens, z.B. eines Erythrozyten, gebunden und über ein heterobifunktionelles Kopplungsreagenz an einen zweiten Antikörper gekoppelt wird, der spezifisch für das nachzuweisende Antigen ist. Ein indirekter Agglutinationstest sowie eine indirekte Bindung des zu bestimmenden Analyten an die Membran eines Erythrozyten wird nicht beschrieben.

Lapierre et al. [Transfusion 30 (1990), 109; FR-A-2 577 321 (FR-A 85 02010)] haben über eine neue Methode in der Blutgruppenserologie berichtet, bei der die Agglutinationsreaktionen in Mikroröhrchen durchgeführt werden, die mit einer Gelsuspension gefüllt sind. Nach einer Sedimentation befinden sich die Agglutinate auf dem Gel oder sind bei schwachen Reaktionen innerhalb des Gels verteilt. Bei Ausbleiben der Agglutination sammeln sich die Zellen am Boden des Röhrchens. Dieses Verfahren ist einfach, sehr empfindlich und wenig störanfällig. Die Ergebnisse sich gut abzulesen und leicht interpretierbar.

Eine Anwendung dieser obigen Gelmethode auf Reaktionen, bei denen Antigene oder antikörper an fixierte Erythrozyten gebunden sind, ist- jedoch nicht möglich. Die Ursache hierfür ist, daß die Erythrozytenmembran nach dem Fixierprozeß nicht mehr verformbar und die fixierten Erythrozyten nicht mehr die Lücken zwischen einzelnen Gelpartikeln passieren können, wodurch eine Unterscheidung zwischen positiver und negativer Reaktion in der Praxis nicht durchführbar ist.

Dieses Problem konnte auch durch eine schwächere Fixierung der Erythrozyten (EP-A 0 305 337) nur teilweise behoben werden, da sich die Reaktion nur schwer steuern läßt. Außerdem wurde festgestellt, daß sich die behandelten Zellen bereits nach kurzer Zeit verändern, indem sie entweder lysieren oder unverformbar werden. Auch eine reine Absorption oder die Vorbehandlung von Zellen mit Chrom-(III) chlorid zeigte keinen oder nur einen sehr geringen Erfolg.

Die Aufgabe der vorliegenden Erfindung bestand somit darin, ein Verfahren zur Bestimmung eines Analyten, d.h. eines Antigens oder Antikörpers durch Agglutination von Erythrozyten bereitzustellen, bei dem die Nachteile des Standes der Technik mindestens teilweise beseitigt werden.

Die erfindungsgemäße Aufgabe wird gelöst durch ein Verfahren zur Bestimmung eines Analyten in einer wäßrigen Probelösung durch Agglutination von nicht-fixierten Erythrozyten, welches dadurch gekennzeichnet ist, daß man
(a) Erythrozyten mit einem oder mehreren nicht zur vorzeitigen Agglutination führenden Antikörpern oder/und Antikörperderivaten inkubiert, wobei die Antikörper oder/und Antikörperderivate gegen ein oder mehrere Antigene auf der Erythrozytenmembran gerichtet sind und wobei die Antikörper oder/und Antikörperderivate vor oder nach der Inkubation mit den Erythrozyten an einen ersten Bindepartner eines hochaffinen Bindepaares gekoppelt werden,
(b) einen zweiten Bindepartner des hochaffinen Bindepaares mit den behandelten Erythrozyten aus (a) inkubiert, wobei der zweite Bindepartner derart gewählt wird, daß er pro Molekül mehr als eine Bindungsstelle für den ersten Bindepartner aufweist,
(c) den ersten Bindepartner des hochaffinen Bindepaares, der mit dem Analyten oder/und einer antigen aktiven Determinante des Analyten gekoppelt ist, mit den behandelten Erythrozyten aus (b) inkubiert,
(d) eine Probelösung, die mit einem gegen den Analyten gerichteten Antikörper oder Antikörperderivat behandelt worden ist, mit den behandelten Erythrozyten aus (c) inkubiert und
(e) anhand des Auftretens bzw. Ausbleibens einer Agglutinationsreaktion das Fehlen bzw. das Vorhandensein des Analyten in der Probelösung bestimmt.

Überraschenderweise wurde festgestellt, daß Antikörper oder/und Antikörperderivate, die nicht zur vorzeitigen Agglutination von Erythrozyten führen, zur Verbrückung von nichtfixierten Erythrozyten geeignet sind. Dabei sind prinzipiell eine Vielzahl verschiedener Antikörpertypen geeignet, jedoch nicht solche Antikörper mit einer konstanten IgM-Domäne. Vorzugsweise verwendet man Antikörper der Klasse IgG oder Antikörperderivate, wie etwa Fab oder F(ab)₂-Fragmente.

Die oben genannten Antikörper sind gegen Antigene auf der Erythrozytenmembran gerichtet. Beispiele hierfür sind Anti-D-, Anti-C-, Anti-E-, Anti-c-, Anti-e-, Anti-M- oder Anti-N-Antikörper. Vorzugsweise verwendet man Anti-D-Antikörper. Zur Verstärkung der Reaktion können auch mehrere dieser Antikörper gleichzeitig eingesetzt werden. Als besonders vorteilhaft hat sich die Verwendung von humanen Antikörper (bzw. von Antikörpern mit einer humanen konstanten Domäne) erwiesen, da es beim Einsatz von Antikörpern mit einer nicht humanen konstanten Domäne zu falsch spezies-spezifischen Reaktionen kommen kann.

Antikörper gegen Erythrozyten sind kommerziell erhältlich oder auf einfache Weise herstellbar. Eine Reinigung kann, sofern erforderlich, nach bekannten Verfahren erfolgen. IgG-Antikörper werden z.B. durch Ammoniumsulfatfällung und lenchromatographie gereinigt. Besonders bevorzugt verwendet man monoklonale Antikörper, da diese in weitgehend gereinigter Form vorliegen. Solche Antikörper können z.B. von Bioscott UK, Monocarb Schweden, Diagast Frankreich usw. bezogen werden.

Der gegen das Antigen auf der Erythrozytenmembran gerichtete Antikörper wird entweder vor oder nach der Inkubation mit den Erythrozyten an einen ersten Bindepartner eines hochaffinen Bindepaares gekoppelt. Der Begriff "hochaffines Bindepaar" bedeutet zwei Substanzen, d.h. den ersten und den zweiten Bindepartner, die in der Lage sind, miteinander eine nichtkovalente Bindung mit einer Bindungskonstante von mindestens 10⁻⁸ Mol/l einzugehen. Als besonders günstig hat sich die Verwendung des Streptavidin- bzw. Avidin-Biotin-Systems erwiesen, wobei man anstelle von Biotin auch ein entsprechendes Biotinderivat verwenden kann. Ein weiteres Beispiel für ein geeignetes Bindepaar ist ein Hapten, d.h. ein niedermolekulares Antigen, wie etwa Fluorescein und ein dagegen gerichteter spezifischer Antikörper.

Die Verwendung des Biotin/Streptavidin-Systems ist besonders bevorzugt, da Streptavidin 4 Bindungsstellen für Biotin aufweist, so daß sich die Zahl der gekoppelten Antigene bzw. Antikörper auf das dreifache erhöhen läßt und überdies die Herstellung der Reagenzien sehr einfach ist, da alle Reaktionsschritte an einer festen Phase erfolgen können. Anstelle von Streptavidin oder Avidin können auch Derivate davon, wie etwa Avidinsuccinyl verwendet werden.

Schritt (a) des erfindungsgemäßen Verfahrens kann auf zwei unterschiedliche Arten durchgeführt werden, gemäß einer ersten Ausführungsform werden die Erythrozyten zuerst mit Antikörpern oder/und Antikörperderivaten inkubiert, die zuvor bereits an den ersten Bindepartner des hochaffinen Bindepaares gekoppelt worden sind. Gemäß einer zweiten und besonders bevorzugten Ausführungsform inkubiert man zuerst die Erythrozyten mit nicht-gekoppelten Antikörpern oder/und Antikörperderivaten und führt erst dann die Kopplung der Antikörper oder/und Antikörperderivate an den ersten Bindepartner des hochaffinen Bindepaares durch.

Die Zugabe des zweiten Bindepartners in Schritt (b) des erfindungsgemäßen Verfahrens erfolgt vorzugsweise so, daß der erste Bindepartner und der zweite Bindepartner in einem molaren Verhältnis von etwa 1:1 vorliegen. Die Zugabe des Analytgekoppelten ersten Bindepartners in Schritt (c) des erfindungsgemäßen Verfahrens erfolgt vorzugsweise so, daß alle freien Bindungsstellen des zweiten Bindepartners besetzt werden. Im Falle des Streptavidin/Biotin-Systems bedeutet dies ein molares Verhältnis von ca. 1:3 (Streptavidin : biotinylierter Analyt).

Das erfindungsgemäße Verfahren dient zur Bestimmung eines Analyten in einer wäßrigen Probelösung. Unter den Begriff Analyt fallen alle durch eine immunologische Antigen-Antikörper-Reaktion bestimmbaren Substanzen. Eine bevorzugte Anwendung für das erfindungsgemäße Verfahren ist jedoch die Durchführung eines Schwangerschaftstests, wobei man z.B. HCG als Analyten bestimmt. Es ist jedoch klar, daß auch andere Analyten, die bei der Erkennung von Krankheiten, insbesondere Hepatitis, AIDS, Syphilis oder Hormonstörungen, eine Rolle spielen, bestimmt werden können. Ferner ist beispielsweise auch ein Nachweis von Medikamenten möglich.

Durch das erfindungsgemäße Verfahren lassen sich auch mehrere verschiedene Analyten gleichzeitig bestimmen, so daß Suchtests für Infektionskrankheiten sehr stark vereinfacht werden können.

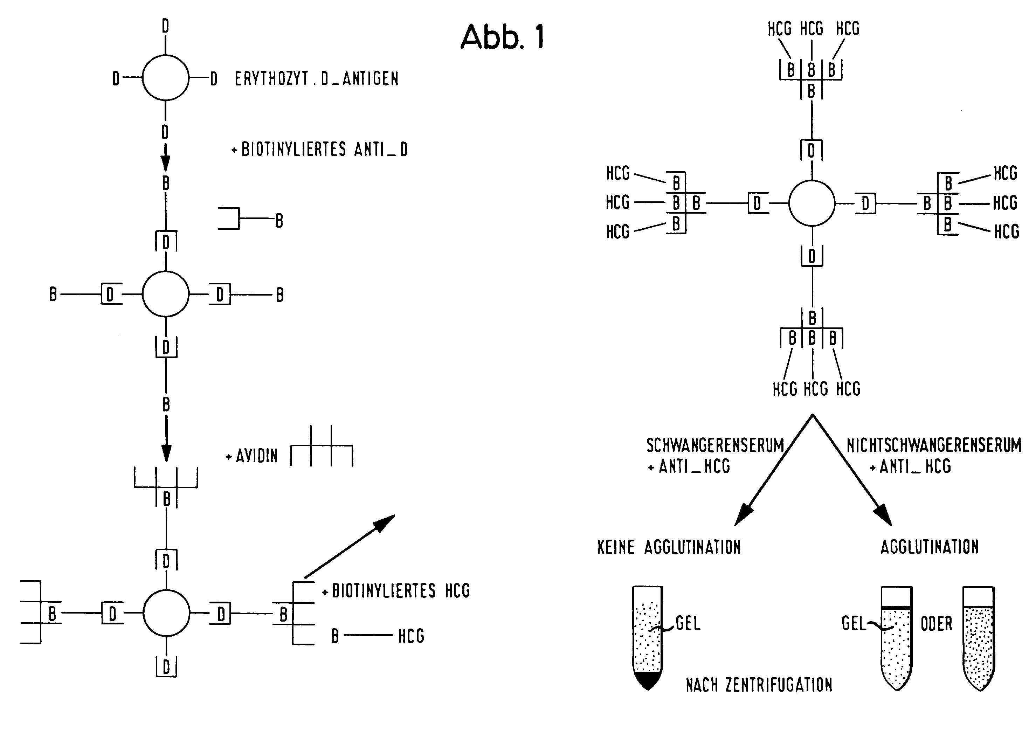

Das Verfahren soll im folgenden am Beispiel eines Schwangerschaftstests näher erläutert werden. Der Ablauf der einzelnen Verfahrensschritte ist schematisch auch in Figur 1 dargestellt. Zuerst wird humanes Blut (z.B. Blutgruppe 0, Rh (D) positiv) mit einem biotinylierten Antikörper (Anti-D) vom Typ IgG versetzt, der gegen das Oberflächenantigen D von Erythrozyten gerichtet ist. Die jeweils erforderliche Menge des Antikörpers hängt von der Affinität des Antikörpers an das Antigen ab. Nach Inkubation und einem Waschschritt wird Streptavidin bzw. Avidin zugegeben und bindet daraufhin mit einer seiner vier Bindungsstellen an den biotinylierten Antikörper. Vorzugsweise erfolgt die Zugabe so, daß das molare Verhältnis von Streptavidin zu Biotin ca. 1:1 ist. Nach einem weiteren Waschen werden die freien Bindungsstellen des Streptavidins bzw. Avidins mit dem biotinylierten Antigen, dem Schwangerschaftshormon HCG belegt, das somit über den Biotin-Avidin-Komplex an den Antikörper gebunden wird. Das molare Verhältnis von Avidin zum biotinylierten Analyten ist besonders bevorzugt 1:3. Der Antikörper wiederum vermittelt die Bindung an die Erythrozyten. Nach Waschen und Verdünnen erhält man ein gebrauchsfertiges Erythrozytenreagenz.

Es ist andererseits aber auch möglich, zuerst den Antikörper mit den Erythrozyten reagieren zu lassen und nach dem Waschen der Zellen den an die Erythrozyten gebundenen Antikörper mit Hilfe eines reaktiven Biotinderivats (z.B. Biotin-N-hydroxysuccinimidester) zu biotinylieren.

Wenn man das Erythrozytenreagenz mit Patientenserum und Anti-HCG-Antikörpern versetzt, so beobachtet man eine Agglutination, sofern das Serum kein HCG enthält. Bei Seren von Schwangeren, die HCG enthalten, wird die Agglutination inhibiert. Für die Auswertung dieses Tests hat sich insbesondere das in der FR-A-2 577 321 beschriebene Gel-Test-System als günstig erwiesen. Mit diesem System kann eine im Vergleich zu herkömmliche Tests um das 25-fach gesteigerte Empfindlichkeit erreicht werden. Die zur Neutralisierung des Analyten erforderliche Antiköpermenge kann von einem Fachmann ohne weiteres durch einfache Versuche (entsprechend bekannten Agglutinationstests) ermittelt werden.

Ein weiterer Gegenstand der vorliegenden Erfindung ist auch ein Reagenz zur Bestimmung eines Analyten in einer wäßrigen Probelösung durch Agglutination von nicht-fixierten Erythrozyten, welches dadurch gekennzeichnet ist, daß es Erythrozyten enthält, an deren Membran ein oder mehrere, nicht zur vorzeitigen Agglutination führende, gegen ein oder mehrere Antigene auf der Erythrozytenmembran gerichtete Antikörper oder/und Antikörperderivate gebunden sind, wobei ein erster Bindepartner eines hochaffinen Bindepaares kovalent an die Antikörper oder/und Antikörperderivate gekoppelt ist und wobei an den ersten Bindepartner ein zweiter Bindepartner des hochaffinen Bindepaares gebunden ist, wobei der zweite Bindepartner pro Molekül mehr als eine Bindungsstelle für den ersten Bindepartner aufweist.

Das erfindungsgemäße Reagenz enthält den zweiten Bindepartner des hochaffinen Bindepaares an den ersten Bindepartner gebunden, wobei der zweite Bindepartner pro Molekül mehr als eine Bindungsstelle für den ersten Bindepartner, besonders bevorzugt vier Bindungsstellen aufweist. Dabei sind die Bindungsstellen des ersten Bindepartners günstigerweise im wesentlichen quantitativ belegt. Weiterhin bevorzugt ist, daß an den zweiten Bindepartner wiederum der erste Bindepartner des hochaffinen Bindepaares gebunden ist, der mit dem Analyten oder/und einer antigen aktiven Determinante des Analyten gekoppelt ist.
Das erfindungsgemäße Reagenz ist in gebrauchsfertigem Zustand ungefähr 2 Monate bei 4°C haltbar, insbesondere wenn es in Form einer Suspension vorliegt.

Weiterhin ein Gegenstand der vorliegenden Erfindung ist auch ein Reagenzienkit, der ein erfindungsgemäßes Reagenz und getrennt davon einen gegen den zu bestimmenden Analyten gerichteteten Antiköper oder ein entsprechendes Antiköperderivat enthält. Vorzugsweise enthält der erfindungsgemäße Reagenzienkit weiterhin mit einer Gelsuspension gefüllte Testgefäße (z.B. zur Zentrifugation in einer Mikrozentrifuge geeignete Röhrchen).

Die vorliegende Erfindung soll weiterhin durch die folgenden Beispiele in Verbindung mit Abbildung 1 verdeutlicht werden. Abbildung 1 zeigt:
die Agglutination von nicht-fixierten Erythrozyten am Beispiel eines Schwangerschaftstests

### Beispiel 1

### Indirekter Schwangerschaftstest

### 1. Herstellung von biotinyliertem Anti-D

1 ml Anti-D (IgG, 100 µg/ml) in 0,1 mol/l NaHCO₃, pH 8,5, wird mit 12 µl Biotin-N-Hydroxysuccinimidester (1 mg/ml in DMSO, SPA Milano) versetzt. Die Mischung wird 72 Stunden bei 4°C stehengelassen und anschließend gegen PBS (Phosphat-gepufferte Salzlösung) dialysiert.

### 2. Herstellung von biotinyliertem HCG

1 ml HCG (0,1 mg/ml 3000 U/mg) in 0,1 mol/l NaHCO₃, pH 8,5, wird mit 12 µl Biotin-N-Hydroxysuccinimidester (1 mg/ml in DMSO) versetzt. Die Mischung wird 72 Stunden bei 4°C stehengelassen und anschließend gegen PBS dialysiert.

### 3. Beladung der Erythrozyten mit biotinyliertem Anti-D-Antikörper

Frische humane Erythrozyten der Blutgruppe 0, Rh (D) positiv werden mit 0,9 % NaCl auf eine Konzentration von 10 % verdünnt. 5 ml dieser Suspension werden mit 0,5 ml biotinyliertem Anti-D versetzt. Nach einer Inkubation von einer Stunde bei Raumtemperatur wird dreimal mit 0,9 % NaCl gewaschen und die Zellen in 5 ml ID-CellStab (Stabilisier-Lösung, DiaMed Murten) suspendiert.

### 4. Reaktion der biotinylierten Zellen mit Avidin

1 ml Avidinsuccinyl 6,93 U/mg (SPA Milano) werden in 70 µl PBS gelöst. Davon werden 60 µl zu 5 ml der unter (3) beschriebenen Erythrozytensuspension gegeben. Nach einer Stunde Inkubation bei Raumtemperatur wird dreimal mit 0,9 % NaCl gewa-schen und das Sediment in ID-CellStab suspendiert.

### 5. Reaktion der avidinierten Zellen mit HCG

5 ml der unter (4) beschriebenen Erythrozyensuspension werden mit 2,5 ml biotinyliertem HCG (0,1 mg/ml) zur Reaktion gebracht. Nach einer Stunde Inkubation bei Raumtemperatur wird dreimal mit 0,9 % NaCl gewaschen und eine ca. 0,8 % Suspension mit ID-CellStab hergestellt. Das so hergestellte Reagenz ist gebrauchsfertig und ungefähr zwei Monate bie 4°C haltbar.

### 6. Testdurchführung

50 µl der mit HCG beladenen Erythrozyten werden in vorgefertigte mit einer Gelsuspension vorgefüllte Mikroröhrchen (ID-Karte, DiaMed Murten) gegeben. Anschließend werden 25 µl Patientenserum und 25 µl Anti-HCG (die richtige Verdünnung des Antikörpers wird vorher ermittelt) zugegeben. (Um Kreuzreaktionen mit LH oder FSH zu vermeiden, ist es vorteilhaft, als Antikörper Anti-B-HCG einzusetzen).

Nach 10 bis 15 Minuten Inkubation bei Raumtemperatur wird 10 Minuten bei ca. 910 U/min in eier für die Mikroröhrchen in der ID-Karte geeigneten Zentrifuge (ID-Zentrifuge, DiaMed Murten) zentrifugiert.

### 7. Interpretation der Ergebnisse

### a) positives Resultat

Der Antikörper hat mit dem HCG im Serum reagiert und ist somit neutralisiert worden. Deshalb kann er nicht mit dem HCG auf den Erythrozyten reagieren. Die nicht-agglutinierten Zellen sammeln sich nach dem Zentrifugieren (Sedimentieren) auf dem Boden des Mikroröhrchens und sind als roter Knopf sichtbar.

### b) negatives Resultat

Der Antikörper reagiert mit dem HCG auf den Erythrozyten. Die Agglutinate liegen nach der Zentrifugation (Sedimentation) je nach der Stärke der Reaktion auf dem Gel oder im Gel verteilt.

### 8. Empfindlichkeit des Tests

Mit dem 2. Int. HCG-Standard der WHO wurde eine Verdünnungsreihe in HCG-negativem Serum gemacht. Die Empfindlichkeit lag bei 25 bis 50 U/l und entspricht damit den im Handel erhältlichen ELISA-Tests der neuesten Generation.

### 9. Richtigkeit des Tests

Jeweils 500 Seren von Schwangeren und Nichtschwangeren wurden im Vergleich mit einem im Handel erhältlichen Schwangerschaftstest (Hoffmann LaRoche, Empfindlichkeit 50 U/l) untersucht. Die Ergebnisse stimmten 100 % überein.

### Beispiel 2

### Biotinylierung von Anti-D an fester Phase (d.h. an der Erythrozytenoberfläche)

### 1. Beladung der Erythrozyten mit Anti-D

Frische Erythrozyten der Blutgruppe 0, Rh (D) positiv, werden mit 0,9 % NaCl auf 10 % verdünnt. Zu 5 ml dieser Suspension werden 0,5 ml Anti-D-Serum (100 µg/ml) gegeben. Nach einer Stunde Inkubation bei Raumtemperatur wird dreimal mit 0,9 % NaCl gewaschen und die Zellen in 5 ml in einer isotonischen Lösung 0,1 mol/l NaHCO₃, pH 8,5, suspendiert.

### 2. Kopplung von Biotin an das Anti-D

1 mg Biotin-N-Hydroxysuccinimidester (SPA Milano) wird in 1 ml DMSO gelöst. Davon werden 6 µl zu 0,5 ml der unter (1) beschriebenen Erythrozytensuspension gegeben. Nach 24 Stunden Inkubation bei 4°C wird dreimal mit 0,9 % NaCl gewaschen und die Zellen mit 5 ml ID-CellStab (DiaMed Murten) resuspendiert.

3. Die weiteren Reaktionen erfolgen wie im Beispiel 1 erläutert.

## Patentansprüche

1. Verfahren zur Bestimmung eines Analyten in einer wäßrigen Probelösung durch Agglutination von nicht-fixierten Erythrozyten,
**dadurch gekennzeichnet,**
daß man
(a) Erythrozyten mit einem oder mehreren nicht zur vorzeitigen Agglutination führenden Antikörpern oder/und Antikörperderivaten inkubiert, wobei die Antikörper oder/und Antikörperderivate gegen ein oder mehrere Antigene auf der Erythrozytenmembran gerichtet sind und wobei die Antikörper oder/und Antikörperderivate vor oder nach der Inkubation mit den Erythrozyten an einen ersten Bindepartner eines hochaffinen Bindepaares gekoppelt werden,
(b) einen zweiten Bindepartner des hochaffinen Bindepaares mit den behandelten Erythrozyten aus (a) inkubiert, wobei der zweite Bindepartner derart gewählt wird, daß er pro Molekül mehr als eine Bindungsstelle für den ersten Bindepartner aufweist,
(c) den ersten Bindepartner des hochaffinen Bindepaares, der mit dem Analyten oder/und einer antigen aktiven Determinante des Analyten gekoppelt ist, mit den behandelten Erythrozyten aus (b) inkubiert,
(d) eine Probelösung, die mit einem gegen den Analyten gerichteten Antikörper oder Antikörperderivat behandelt worden ist, mit den behandelten Erythrozyten aus (c) inkubiert und
(e) anhand des Auftretens bzw. Ausbleibens einer Agglutinationsreaktion das Fehlen bzw. das Vorhandensein des Analyten in der Probelösung bestimmt.

2. Verfahren nach Anspruch 1,
**dadurch gekennzeichnet,**
daß man in Schritt (a) Erythrozyten mit Antikörpern oder/und Antikörperderivaten inkubiert, die zuvor an den ersten Bindepartner eines hochaffinen Bindepaares gekoppelt worden sind.

3. Verfahren nach Anspruch 1,
**dadurch gekennzeichnet,**
daß man in Schritt (a) zuerst Erythrozyten mit nichtgekoppelten Antikörpern oder/und Antikörperderivaten inkubiert und dann die Kopplung der Antikörper oder/und Antikörperderivate an den ersten Bindepartner eines hochaffinen Bindepaares an fester Phase durchführt.

4. Verfahren nach einem der Ansprüche 1 bis 3,
**dadurch gekennzeichnet,**
daß man Antikörper der Klasse IgG als Antikörper verwendet, die gegen Antigene auf der Erythrozytenmembran gerichtet sind.

5. Verfahren nach einem der Ansprüche 1 bis 4,
**dadurch gekennzeichnet,**
daß man Antikörper humanen Ursprungs als Antikörper verwendet, die gegen Antigene auf der Erythrozytenmembran gerichtet sind.

6. Verfahren nach einem der Ansprüche 1 bis 5,
**dadurch gekennzeichnet,**
daß man Biotin oder ein Biotinderivat als ersten Bindepartner und Avidin oder Streptavidin oder Derivate davon als zweiten Bindepartner verwendet.

7. Verfahren nach einem der Ansprüche 1 bis 6,
**dadurch gekennzeichnet**,
daß man das Auftreten bzw. Ausbleiben einer Agglutinationsreaktion in einem Gel-Sedimentationstest bestimmt.

8. Verfahren nach einem der Ansprüche 1 bis 7,
**dadurch gekennzeichnet**,
daß man Anti-D Antikörper als Antikörper verwendet, die gegen Antigene auf der Erythrozytenmembran gerichtet sind.

9. Verfahren nach einem der Ansprüche 1 bis 8,
**dadurch gekennzeichnet**,
daß man einen Schwangerschaftstest durchführt.

10. Verfahren nach Anspruch 9,
**dadurch gekennzeichnet**,
daß man HCG als Analyten bestimmt.

11. Verfahren nach einem der Ansprüche 1 bis 8,
**dadurch gekennzeichnet**,
daß man einen Nachweis von Analyten durchführt, die bei der Erkennung von Krankheiten, insbesondere Hepatitis, AIDS, Syphilis oder Hormonstörungen eine Rolle spielen.

12. Reagenz zur Bestimmung eines Analyten in einer wäßrigen Probelösung durch Agglutination von nicht-fixierten Erythrozyten,
**dadurch gekennzeichnet**,
daß es Erythrozyten enthält, an deren Membran ein oder mehrere, nicht zur vorzeitigen Agglutination führende, gegen ein oder mehrere Antigene auf der Erythrozytenmembran gerichtete Antikörper oder/und Antikörperderivate gebunden sind, wobei ein erster Bindepartner eines hochaffinen Bindepaares kovalent an die Antikörper oder/und Antikörperderivate gekoppelt ist und wobei an den ersten Bindepartner ein zweiter Bindepartner des hochaffinen Bindepaares gebunden ist, wobei der zweite Bindepartner pro Molekül mehr als eine Bindungsstelle für den ersten Bindepartner aufweist.

13. Reagenz nach Anspruch 12,
**dadurch gekennzeichnet**,
daß weiterhin an den zweiten Bindepartner der erste Bindepartner des hochaffinen Bindepaares gebunden ist, der mit dem Analyten oder/und einer antigen aktiven Determinante des Analyten gekoppelt ist.

14. Reagenz nach Anspruch 12 oder 13,
**dadurch gekennzeichnet**,
daß der zweite Bindepartner pro Molekül vier Bindungsstellen für den ersten Bindepartner aufweist.

15. Reagenz nach einem der Ansprüche 12 bis 14,
**dadurch gekennzeichnet**,
daß es in Form einer Suspension vorliegt.

16. Reagenzienkit,
**dadurch gekennzeichnet**,
daß er ein Reagenz nach einem der Ansprüche 12 bis 15 und getrennt davon einen gegen den zu bestimmenden Analyten gerichteten Antikörper oder ein entsprechendes Antikörperderivat enthält.

17. Reagenzienkit nach Anspruch 16,
**dadurch gekennzeichnet**,
daß er weiterhin mit einer Gelsuspension gefüllte Testgefäße enthält.

## Claims

1. Method for the determination of an analyte in an aqueous sample solution by agglutination of non-fixed erythrocytes,
**wherein**
(a) erythrocytes are incubated with one or several antibodies or/and antibody derivatives which do not lead to premature agglutination whereby the antibodies or/and antibody derivatives are directed towards one or several antigens on the erythrocyte membrane and whereby before or after incubation with the erythrocytes the antibodies or/and antibody derivatives are coupled to a first binding partner of a high affinity binding pair,
(b) a second binding partner of the high affinity binding pair is incubated with the treated erythrocytes from (a) whereby the second binding partner is chosen such that it has more than one binding site per molecule for the first binding partner,
(c) the first binding partner of the high affinity binding pair which is coupled with the analyte or/and an antigen-active determinant of the analyte is incubated with the treated erythrocytes from (b),
(d) a sample solution which has been treated with an antibody or antibody derivative directed towards the analyte is incubated with the treated erythrocytes from (c) and
(e) the absence or presence of the analyte in the sample solution is determined on the basis of the occurrence or absence of an agglutination reaction.

2. Method as claimed in claim 1,
**wherein**
in step (a) erythrocytes are incubated with antibodies or/and antibody derivatives which have been previously coupled to the first binding partner of a high affinity binding pair.

3. Method as claimed in claim 1,
**wherein**
in step (a) the erythrocytes are firstly incubated with non-coupled antibodies or/and antibody derivatives and then the coupling of the antibodies or/and antibody derivatives to the first binding partner of a high affinity binding pair is carried out on a solid phase.

4. Method as claimed in one of the claims 1 to 3,
**wherein**
antibodies of the IgG class which are directed towards antigens on the erythrocyte membrane are used as antibodies.

5. Method as claimed in one of the claims 1 to 4,
**wherein**
antibodies of human origin which are directed towards antigens on the erythrocyte membrane are used as antibodies.

6. Method as claimed in one of the claims 1 to 5,
**wherein**
biotin or a biotin derivative is used as the first binding partner and avidin or streptavidin or derivatives thereof is used as the second binding partner.

7. Method as claimed in one of the claims 1 to 6,
**wherein**
the occurrence or absence of an agglutination reaction is determined in a gel sedimentation test.

8. Method as claimed in one of the claims 1 to 7,
**wherein**
anti-D antibodies which are directed towards antigens on the erythrocyte membrane are used as antibodies.

9. Method as claimed in one of the claims 1 to 8,
**wherein**
a pregnancy test is carried out.

10. Method as claimed in claim 9,
**wherein**
HCG is determined as the analyte.

11. Method as claimed in one of the claims 1 to 8,
**wherein**
a test is carried out for analytes which play a role in the detection of diseases in particular of hepatitis, AIDS, syphilis or hormone disorders.

12. Reagent for the determination of an analyte in an aqueous sample solution by agglutination of non-fixed erythrocytes,
**wherein**
it contains erythrocytes on the membrane of which one or several antibodies or/and antibody derivatives directed towards one or several antigens on the erythrocyte membrane which do not lead to a premature agglutination are bound and whereby a first binding partner of a high affinity binding pair is covalently coupled to the antibodies or/and antibody derivatives and whereby a second binding partner of the high affinity binding pair is bound to the first binding partner, the second binding partner having more than one binding site per molecule for the first binding partner.

13. Reagent as claimed in claim 12,
**wherein**
in addition the first binding partner of the high affinity binding pair which is coupled to the analyte or/and an antigen-active determinant of the analyte is coupled to the second binding partner.

14. Reagent as claimed in claim 12 or 13,
**wherein**
the second binding partner has four binding sites per molecule for the first binding partner.

15. Reagent as claimed in one of the claims 12 to 14,
**wherein**
it is present in the form of a suspension.

16. Reagent kit,
**wherein**
it contains a reagent as claimed in one of the claims 12 to 15 and separate from this an antibody or a corresponding antibody derivative which is directed towards the analyte to be determined.

17. Reagent kit as claimed in claim 16,
**wherein**
it additionally contains a test vessel which is filled with a gel suspension.

## Revendications

1. Procédé pour déterminer un analyte dans une solution échantillon aqueuse par agglutination d'érythrocytes non fixés, caractérisé en ce que
(a) on incube des érythrocytes avec un ou plusieurs anticorps et/ou dérivés d'anticorps qui ne conduisent pas à une agglutination prématurée, les anticorps et/ou dérivés d'anticorps étant dirigés contre un ou plusieurs antigènes sur la membrane des érythrocytes et les anticorps et/ou dérivés d'anticorps étant couplés à un premier partenaire de liaison d'une paire de liaison à haute affinité avant ou après l'incubation avec les érythrocytes,
(b) on incube un second partenaire de liaison de la paire de liaison à haute affinité avec les érythrocytes traités provenant de (a), le second partenaire de liaison étant choisi de manière qu'il comporte par molécule plus d'un site de liaison pour le premier partenaire de liaison,
(c) on incube le premier partenaire de liaison de la paire de liaison à haute affinité, qui est couplé à l'analyte et/ou à un déterminant de l'analyte actif du point de vue antigénique, avec les érythrocytes traités provenant de (b),
(d) on incube une solution échantillon qui a été traitée avec un anticorps ou dérivé d'anticorps dirigé contre l'analyte avec les érythrocytes traités provenant de (c) et
(e) on détermine l'absence ou la présence de l'analyte dans la solution échantillon à l'aide de la survenue ou de l'absence d'une réaction d'agglutination.

2. Procédé selon la revendication 1 caractérisé en ce que, dans l'étape (a), on incube des érythrocytes avec des anticorps et/ou des dérivés d'anticorps qui ont été couplés au préalable au premier partenaire de liaison d'une paire de liaison à haute affinité.

3. Procédé selon la revendication 1 caractérisé en ce que, dans l'étape (a), on incube tout d'abord des érythrocytes avec des anticorps et/ou dérivés d'anticorps non couplés puis on réalise le couplage des anticorps et/ou dérivés d'anticorps au premier partenaire de liaison d'une paire de liaison à haute affinité sur une phase solide.

4. Procédé selon l'une des revendications 1 à 3 caractérisé en ce que l'on utilise comme anticorps des anticorps de la classe des IgG qui sont dirigés contre des antigènes sur la membrane des érythrocytes.

5. Procédé selon l'une des revendications 1 à 4 caractérisé en ce que l'on utilise comme anticorps des anticorps d'origine humaine qui sont dirigés contre des antigènes sur la membrane des érythrocytes.

6. Procédé selon l'une des revendications 1 à 5 caractérisé en ce que l'on utilise comme premier partenaire de liaison la biotine ou un dérivé de biotine et comme second partenaire de liaison l'avidine ou la streptavidine ou des dérivés de celles-ci.

7. Procédé selon l'une des revendications 1 à 6 caractérisé en ce que l'on détermine la survenue ou l'absence d'une réaction d'agglutination dans un test de sédimentation sur gel.

8. Procédé selon l'une des revendications 1 à 7 caractérisé en ce que l'on utilise comme anticorps des anticorps anti-D qui sont dirigés contre des antigènes sur la membrane des érythrocytes.

9. Procédé selon l'une des revendications 1 à 8 caractérisé en ce que l'on met en oeuvre un test de grossesse.

10. Procédé selon la revendication 9 caractérisé en ce que l'on détermine HCG comme analyte.

11. Procédé selon l'une des revendications 1 à 8 caractérisé en ce que l'on réalise une mise en évidence d'analytes qui jouent un rôle dans l'identification de maladies, en particulier de l'hépatite, du SIDA, de la syphilis ou des troubles hormonaux.

12. Réactif pour la détermination d'un analyte dans une solution échantillon aqueuse par agglutination d'érythrocytes non fixés caractérisé en ce qu'il contient des érythrocytes à la membrane desquels sont liés un ou plusieurs anticorps et/ou dérivés d'anticorps dirigés contre un ou plusieurs antigènes sur la membrane des érythrocytes, qui ne conduisent pas à une agglutination prématurée, un premier partenaire de liaison d'une paire de liaison à haute affinité étant couplé de manière covalente aux anticorps et/ou dérivés d'anticorps et un second partenaire de liaison de la paire de liaison à haute affinité étant lié au premier partenaire de liaison, le second partenaire de liaison présentant par molécule plus d'un site de liaison pour le premier partenaire de liaison.

13. Réactif selon la revendication 12 caractérisé en ce que le premier partenaire de liaison de la paire de liaison à haute affinité qui est couplé à l'analyte et/ou à un déterminant de l'analyte actif du point de vue antigénique est lié en outre au second partenaire de liaison.

14. Réactif selon la revendication 12 ou 13 caractérisé en ce que le second partenaire de liaison présente par molécule quatre sites de liaison pour le premier partenaire de liaison.

15. Réactif selon l'une des revendications 12 à 14 caractérisé en ce qu'il est sous forme d'une suspension.

16. Trousse de réactifs caractérisée en ce qu'elle contient un réactif selon l'une des revendications 12 à 15 et, séparé de celui-ci, un anticorps dirigé contre l'analyte à déterminer ou un dérivé d'anticorps correspondant.

17. Trousse de réactifs selon la revendication 16 caractérisée en ce qu'elle contient en outre des récipients a essai remplis d'une suspension de gel.
